# EUROPÄISCHE PATENTSCHRIFT

(11) **EP 0 480 551 B1**
(45) Veröffentlichungstag und Bekanntmachung des Hinweises auf die Patenterteilung: **10.01.1996**
(21) Anmeldenummer: 91250274.7
(22) Anmeldetag: 08.10.1991
(51) Int. Cl.: A61F 2/34

(54) **Einschraubbare Hüftgelenkpfanne und Verfahren zu deren Herstellung**
Screw-on acetabular prosthesis and method of manufacturing the same
Prothèse acétabulaire à visser et son procédé de fabrication

(30) Priorität: 09.10.1990 DE 4031926
(43) Veröffentlichungstag der Anmeldung: 15.04.1992
(73) Patentinhaber: Johnson & Johnson Professional Products GmbH, D-22848 Norderstedt (DE)
(72) Erfinder: Hörmansdörfer, Gerd, 31303 Burgdorf-Beinhorn (DE)
(74) Vertreter: UEXKÜLL & STOLBERG

(56) Entgegenhaltungen:
- EP-A- 0 237 751
- EP-A- 0 277 511
- FR-A- 2 548 012

## Beschreibung

Die Erfindung betrifft eine künstliche einschraubbare Hüftgelenkpfanne für die Verwendung in der Humanmedizin sowie ein Verfahren zu deren Herstellung.

Hüftgelenkpfannen sind Komponenten des künstlichen Gelenkersatzes beim Menschen. Die Hüftgelenkendoprothetik ist, ebenso wie andere Bereiche der medizinischen Technik auch, von ihrem Anbeginn an von einer stetigen Entwicklung gekennzeichnet, angetrieben von dem Bestreben, die natürlichen Funktionen möglichst perfekt und dauerhaft nachzugestalten, um dem Patienten in bestmöglicher Weise zu dienen. Dabei war man auch vor Rückschlägen nicht gefeit, wenn es darum ging, die optimale Geometrie, den am besten geeigneten Werkstoff, oder auch die sicherste Operationsmethode zu finden. Aufgrund der in heutiger Zeit stark erhöhten Lebenserwartung des Menschen ist der Ersatz von Hüftgelenken nicht nur quantitativ angestiegen, sondern sind die Anforderungen auch qualitativ höher geworden, weil von doch wesentlich längeren Beanspruchungszeiträumen auszugehen ist. Vor diesem Hintergrund sind auch heute die dem Stand der Technik entsprechenden Systeme noch nicht vollständig optimiert. Die Erfindung soll hier in einem Teilbereich einen Beitrag zu einer Verbesserung der medizinischen Technik leisten.

Im Prinzip lassen sich derartige Hüftgelenkpfannen in zwei wesentliche Gruppen aufteilen, zementierbare und zementlose Hüftgelenkpfannen. Davon findet die zementlose Hilftgelenkpfanne auch im höchsten Alter und in Verbindung mit einem zementfreien Schaft immer häufigere Anwendung. Von dieser Bauart ist jene am weitesten verbreitet, welche aus einem metallischen Einschraubkörper und einem Inlett aus Kunststoff besteht. In einem solchen Fall ist der Einschraubkörper mit einem außenliegenden Gewinde versehen. Man unterscheidet zusätzlich selbstschneidende Gewinde von solchen, bei denen im Acetabulum (Becken) das Gewinde zuerst vorgeschnitten werden muß. Die genannten Bauarten lassen sich ferner gemäß unterschiedlicher Außenformen weiter aufgliedern in kegelige, sphärische, kegelig-sphärische, und relativ unbedeutende zylindrische Querschnitte.

Es ist mittlerweile bekannt, daß bei einer Implantation einer Hüftgelenkendoprothese ein dauerhafter Erfolg nicht immer garantiert werden kann. In Bezug auf die Hüftgelenkpfanne werden häufiger Lockerungen beobachtet, welche zu entzündlichen Vorgängen führen und Reoperationen erfordern. Eine sehr stabile Primärfixation der Hüftgelenkpfanne wird heute als eine sehr wesentliche Vorkehrung gegen derartige Lockerungen angesehen.

Sehr gute Primärfixationen lassen sich theoretisch mit konischen Hüftgelenkpfannen erzielen, wenn von einem exakt winkeltreuen Einschrauben der Pfanne in die Ausfräsung des Beckens ausgegangen wird. In der Praxis sind jedoch meist niedrigere Werte anzusetzen, weil bereits sehr kleine Winkelfehler lediglich zu linienförmigen Berührungen zwischen dem tragenden Knochen und dem Kegelmantel der Pfanne führen. Konische Pfannen haben ferner den Nachteil, daß sie große Fräsvolumina erfordern, da ihre äußere Geometrie nicht der Anatomie entspricht.

Sphärische Pfannen entsprechen vollständig der anatomischen Form. Für die Implantation kann das natürliche Knochengewebe weitestgehend erhalten werden. Vorteilhaft wirkt sich hier ferner die Kugel/Kugel-Passung aus, weil dadurch der Einschraubwinkel der Pfanne in die vorbereitete Ausfräsung im Becken keine Auswirkung auf die Verankerungsfestigkeit hat. Sphärische Pfannen leiden unter dem Mangel, daß ihr Gewinde in seinem Grund nicht genau der angestrebten Kugelform folgt. Ein auf dem vom Nenndurchmesser der Pfanne gebildeten Kugelmantel verlaufender Gewindegrund ließe sich zwar mit einem normalen Schraubengewinde (Spitzgewinde mit Dreieckprofil) ohne weiteres realisieren, jedoch ist dieses Gewindeprofil der vorgesehenen Verwendung nicht adäquat. Eine Aufteilung des Gewindeprofils in schmale Gewindezähne und breite Gewinderillen, ähnlich wie z.B. bei Holzschrauben, ist anzustreben, weil durch die breite Basis der knochenseltigen Gewinderippen ein besserer Erhalt der lebenden Knochensubstanz gewährleistet ist und außerdem eine derartige Teilung das Festigkeitsverhältnis der Werkstoffpaarung zwischen Knochen und z.B. Metall berücksichtigt. Daraus ergibt sich die Notwendigkeit, derartige Gewinde mit mehr oder weniger trapezförmigen Werkzeugen herzustellen, deren den Gewindegrund schneidende Schneidkanten einen festen Winkel - in der Regel 0° zur Pfannenachse - besitzen. Der Axialschnitt eines solchen Gewindes zeigt einen treppenstufenartigen Verlauf. Werden die Gewinderillen tief genug geschnitten, um ein vollständiges Eindrehen der Pfanne zu ermöglichen, liegt der Gewindegrund der Pfanne gegenüber den knochenseitigen Gewindespitzen frei, so daß bei entsprechender Belastung die Gefahr des Lockerns besteht. Außerdem wird dadurch eine ungünstig dicke Pfannenwandung erforderlich. Werden die Gewinderillen weniger tief geschnitten, tritt das Phänomen auf, daß die Gewindezähne in Richtung zum Pfannenpol hin immer kleiner werden und gleichzeitig ein immer größer werdender Anteil des Gewindegrundes aus da Kugelmantel herausragt und beim Einschrauben der Pfanne ein Festgehen bewirkt, ehe der Pfannenrücken am Beckengrund zur Anlage kommt. Selbst wenn in einem solchen Fall der verbleibende Hohlraum mit Knochenspänen aufgefüllt wird, sind damit die grundsätzlichen Mängel solch eines Gewindes nicht beseitigt.

Eine Kompromißlösung stellen konisch-sphärische Hüftgelenkpfannen dar, welche einen sphärischen Rücken und konische Flanken besitzen. Allerdings lassen sich hier akzeptable geometrische Übergänge zwischen da konischen und dem sphärischen Bereich lediglich bei sehr kurzen Gewindelängen verwirklichen. Dadurch ist nur eine verminderte Primärfixation gegeben. Verschiedene Konstruktionen versuchen, diesen Mangel dadurch abzustellen, daß in der Pfanne Bohrungen für die zusätzliche Befestigung mittels Knochenschrauben eingebracht sind. Diese Maßnahmen erhöhen nicht nur die Herstellungskosten, sondern auch den operativen Aufwand.

Aus EP-A-0237751 ist eine einschraubbare Hüftgelenkpfanne mit sphärischer äußerer Kontur bekannt, die mit einem selbstschneidenden Gewinde zur zementfreien Verankerung im Acetabulum versehen ist, wobei der Gewindegrund der sphärischen äußeren Kontur weitgehend angeglichen ist. Die Hüftgelenkpfanne wird aus Titan oder einer Titanlegierung gegossen.

Generell besteht die Möglichkeit, sphärische Hüftgelenkpfannen mit dem gewünschten Verlauf des Gewindegrundes im Feingußverfahren herzustellen. Es ist jedoch bekannt, daß sowohl die Festigkeitswerte als auch das dynamische Verhalten von Feingußlegierungen in keinem Fall die entsprechenden Werte geschmiedeter Werkstoffe erreichen. Außerdem ist das Feingußverfahren nur bei sehr großen Stückzahlen wirtschaftlich.

Es bestand daher die Aufgabe, eine einschraubbare Hüftgelenkpfanne mit einem speziellen Gewinde zu entwickeln, dessen Grund dem Verlauf des vom Nenndurchmesser der Pfanne gebildeten Kugelmantels möglichst vollkommen entspricht sowie ein Verfahren zur Herstellung der Hüftgelenkpfanne zur Verfügung zu stellen.

Der erste Teil dieser Aufgabe wird dadurch gelöst, daß der Gewindegrund in seiner Abwicklung aus streifenförmigen, in der Ebene der Abwicklung parallel zueinander verlaufenden Teilfächen besteht, die zwischen sich jeweils eine stumpfwinklige und schräg zur Erstreckung der Abwicklung verlaufende Dachkante ausbilden, wobei diese Teilflächen im Hüftgelenkpfannen-Axialquerschnitt gerade oder konvex gekrümmte Querschnittsbereiche aufweisen, die im Hüftgelenkpfannen-Axialquerschnitt schräg zu anderen Querschnittsbereichen verlaufen, und wobei diese den Gewindegrund bildenden Teilflächen der sphärischen äußeren Kontur der Hüftgelenkpfanne weitestgehend angeglichen sind.

Mit der Erfindung wird eine einschraubbare Hüftgelenkpfanne mit einer wesentlich verbesserten Geometrie des Gewindes zur Verfügung gestellt, die wesentliche Vorteile zur Folge hat. Die erfindungsgemäße Hüftgelenkpfanne erlaubt aufgrund des nahezu perfekt auf dem Kugelmantel nachgebildeten Gewindegrundes einen wirklich formschlüssigen Einbau in das vorbereitete Becken, wobei wegen des besseren Traganteils des Gewindes eine deutlich erhöhte gewindeinduzierte Vorspannung und damit eine verbesserte Primärfixation gegeben ist. Diese wird auch dadurch erhöht, daß das Gewinde der erfindungsgemäßen Hüftgelenkpfanne, anders als bei den bisher bekannten Pfannen, bis weit bis zum Pfannenpol hin heruntergezogen werden kann. Desweiteren führt der großflächige und ohne Lastspitzen über die gesamte Oberfläche der erfindungsgemäßen Hüftgelenkpfanne bestehende Kontakt zum Knochenbett zu einer geringen spezifischen Beanspruchung der Knochenstruktur. Damit kann von einer Senkung der bisherigen Lockerungsrate ausgegangen werden. Günstig ist ferner die Tatsache, daß dar Verlauf des Gewindegrundes die Herstellung sehr dünnwandiger und damit elastischerer Hüftgelenkpfannen erlaubt. Der Einschraubwinkel der Pfanne ist unkritisch und wirkt sich auf die Verankerung im Knochen nicht nachteilig aus, so daß die Implantation auch von einem weniger geübten Operateur problemlos durchführbar ist.

Die obenstehende Aufgabe wird nach der Erfindung weiterhin mittels eines besonderen Herstellungsverfahrens gelöst. Danach wird vorgeschlagen, das Gewinde mit vershiedenen Werkzeugen mit in Bezug auf den Gewindegrund unterschiedlicher Schneidkantengeometrie auf unterschiedlichen Bahnen zu bearbeiten, wobei jeweils der in Richtung zum Mittelpunkt der vom Nenndurchmesser der Hüftgelenkpfanne gebildeten Halbkugel am weitesten vorstehende Punkt der Schneidkante im wesentlichen parallel zu bzw. auf dem Mantel dieser Halbkugel bewegt wird, und wobei die den Gewindegrund bildenden Teilflächen dem Oberflächenverlauf einer dem Nenndurchmesser der Hüftgelenkpfanne entsprechenden Halbkugel möglichst weitgehend angepaßt sind.

Dieser Oberflächenverlauf kann bereits mit zwei verschiedenen Bearbeitungswerkzeugen grob nachgebildet werden, wenn z.B. eines davon dem kleinsten Tangentenwinkel des vom Gewinde überstrichenen Halbkugelbereichs entspricht (also z.B. den Gewindegrund mit 0° schneidet) und das zweite da größten Tangentenwinkel des vom Gewinde überstrichenen Halbkugelbereichs entspricht (in der Praxis Winkel zwischen etwa 30° und 45°).

Ein der Kugelform wesentlich näher kommender Verlauf des Gewindegrundes ist nach der Erfindung mittels mindestens dreier verschiedener Werkzeuge erzielbar, weshalb dieses Verfahren gegenüber dem vorgenannten bevorzugt ist. In diesem Fall schneidet z.B. das erste Werkzeug den Gewindegrund in einem Teilbereich mit da kleinsten Tangentenwinkel des vom Gewinde überstrichenen Halbkugelbsreichs (z.B. 0°), das zweite Werkzeug mit da mittleren Tangentenwinkel des vom Gewinde überstrichenen Halbkugelbereichs (z.B. 20°), und das dritte Werkzeug mit dem größten Tangentenwinkel des vom Gewinde überstrichenen Halbkugelbereichs (z.B. 40°).

Obwohl die Erfindung hier und nachstehend auf gerade Schneidkanten abgestellt ist, umfaßt der vorstehend verwendete Terminus "Schneidkantengeometrie" natürlich auch gekrümmte Schneidkanten. Somit ist es im Rahmen der Erfindung auch möglich, Werkzeuge mit konkav geschliffenen Schneidkanten einzusetzen, wobei der Radius des konkaven Schneidkantenbasens im wesentlichen dem Radius der Hüftgelenkpfanne entspricht. Die Abstufung der Schneidkanten erfolgt dann hier so, daß anstelle des Schneidkantenwinkels der Winkel einer an den jeweils umfänglichen Mittelpunkt der bogenförmigen Schneidkante gelegten Tangente im Rahmen des Tangentenwinkels des vom Gewinde überstrichenen Halbkugelbereichs abgestuft wird.

Eine nahezu perfekte Nachgestaltung der Halbkugelform im Verlauf des Gewindegrundes ist nach der Erfindung mittels eines Verfahrens erzielbar, bei welchem für den Fertigschnitt vier verschiedene Werkzeuge eingesetzt werden. Hier wird bevorzugt vorgeschlagen, den Gewindegrund für die Barbeitung wiederum in nebeneinander liegende und nahezu gleichmäßig gestaffelte Teilbereiche unterschiedlicher Winkel entsprechend dem vom Gewinde überstrichenen Halbkugelbereich aufzugliedern (z. B. 0°, 14°, 28°, 42°). Dabei hängt es dann von den sich in der Praxis am günstigsten erweisenden Schnittbedingungen ab, in welcher Reihenfolge die einzelnen Werkzeuge zum Einsatz gelangen. Nach der Erfindung ist eine noch feinere Untergliederung der Gewindegrundwinkel für die Bearbeitung in fünf oder mehr Schritten machbar, wobei sich jedoch die Frage nach da Kosten/Nutzen-Verhältnis stellt.

Für die den Gewindegrund betreffende winkelmäßige Aufgliederung der Bearbeitungswerkzeuge wird gemäß weiterer Erfindung vorgeschlagen, den jeweiligen Winkelschritt zu den größeren Winkeln hin sukzessive zu verkleinern (Gewindegrund-Teilwinkel z.B. 0°, 12°, 23°, 33°, 42°), um ein Anwachsen der zum Pfannenpol hin sonst zunehmenden Höhe der im Gewindegrund gebildeten Dachkante entgegen zu wirken. Ferner wird als vorteilhaft vorgeschlagen, den kleinsten Gewindegrund-Teilwinkel je nach Breite der Gewinderille statt auf 0° auf z.B. 2° zu setzen, womit sich die oben genannten Gewindegrund-Teilwinkel z.B. auf 2°, 13,5°, 24°, 33,5° und 42° verändern würden.

Bei der spanenden Herstellung der Hüftgelenkpfanne mit einem erfindungsgemäßen Gewinde werden die einzelnen Werkzeuge relativ zum Axialschnitt der Pfanne auf unterschiedlichen Bahnen bewegt, wobei entweder das Werkstück Pfanne rotiert und das Werkzeug in einer Ebene verfahren wird (drehtechnische Herstellung), oder das Werkstück Pfanne feststeht und die Spindel alt dem Werkzeug rotiert, wobei sich Werkstück und Spindel relativ zueinander schraubenlinienförmig bewegen (frästechnische Herstellung), bzw. wobei sowohl das Werkstück Pfanne als auch ein angetriebenes Werkzeug rotiert und dabei das Werkzeug gleichzeitig in einer Ebene verfahren wird (Drehfräsen). Damit dabei im Verlauf des Gewindegrunds die gewünschte halbkugelförmige Kontur erzielt wird, werden die Verfahrbahnen der jeweiligen Werkzeuge so festgelegt, daß ihr jeweils in Richtung zum Mittelpunkt des vom Nenndurchmesser der Hüftgelenkpfanne gebildeten Halbkugelmantels am weitesten vorstehender Punkt der Schneidkante sich im wesentlichen parallel zu, bzw. auf diesem Halbkugelmantel bewegt.

Daraus ergibt sich z.B. für die Bearbeitung mit drei Werkzeugen, daß sich sowohl für das Werkzeug mit dem größten, als auch für das Werkzeug mit dem kleinsten Schneidkantenwinkel die unkorrigierte Bahn, abgesehen von zu berücksichtigenden Parallelverschiebungen, gegenüber einem Kreisbogen nicht ändert, soweit der Tangentenwinkel des vom Gewinde überstrichenen Bereiches mit seinem Minimal- bzw. Maximalwert mit den kleinsten bzw. größten Schneidkantenwinkel übereinstimmt. Das Werkzeug mit dem mittleren Schneidkantenwinkel wird so jedoch auf einer aus zwei Halbbögen zusammengesetzten Bahn bewegt, weil zuerst seine eine Ecke der Schneidkante und dann seine andere Ecke der Schneidkante der Bogenform des vom Nenndurchmesser der Hüftgelenkpfanne gebildeten Halbkugelmantels folgt. Der so hergestellte Gewindegrund kommt dem idealen Verlauf schon sehr nahe.

Zur weiteren Annäherung des Gewindegrundes an den angestrebten Verlauf auf der Oberfläche des Kugelmantels und auch zur Perfektionierung der Gewindeflanken werden mit der Erfindung zusätzliche Korrekturmaßnahmen vorgeschlagen, welche den einzelnen im Prinzip kreisbogenförmigen, bzw. aus Kreisbögen zusammengesetzten Bahnen zusätzlich überlagert werden. Diese Korrekturen sind:
1. Schneidenradiuskorrektur
2. Flankenverschiebung
3. Sehnenkorrektur
4. Dachkantenkorrektur
Die genannte Schneidenradiuskorrektur ergibt sich aus der Eckenverrundung der Werkzeuge. Es sind zur Zeit keine CNC-Steuerungen bekannt, welche entweder beim Gewindedrehen oder auch beim Gewindefräsen die Eckenradien der Werkzeuge berücksichtigen. Dieses Problem wird erfindungsgemäß dadurch beseitigt, daß für die Berechnung der von den einzelnen Werkzeugen zu fahrenden Bahnen als Radius nicht der halbe Nenndurchmesser der Schraubpfanne herangezogen wird, sondern die Summe aus diesem Radius und dem Schneidenradius des Werkzeugs, und gleichzeitig als Bezugspunkt in der Werkzeugbeschreibung der Mittelpunkt des Schneidenradius einer Ecke des Werkzeugs benutzt wird. Für den Aufbau des Programms muß einheitlich für die gesamte Gruppe von Schneidwerkzeugen jeweils eine Werkzeugecke als Bezug festgelegt werden. Es ist im Prinzip gleichgültig, ob dieses die rechte oder linke Ecke ist. Es ist jedoch zu beachten, daß sich für die jeweils zwei Ecken eines Werkzeuges unterschiedliche Bahnen ergeben.

Die erfindungsgemäße Flankenverschiebung soll sicherstellen, daß die beiden Gewindeflanken sauber und ohne rillenartige Markierungen geschnitten werden. Dazu wird vorgeschlagen, das Werkzeug mit dem kleinsten Schneidkantenwinkel (z.B. 0°) mittels der Werkzeugkorrektur geringfügig, z.B. um einen Betrag von 20 µm, in Richtung zum Pfannenäquator hin und gleichzeitig die Konturbeschreibung in Gestalt der abzufahrenden Bahn mit dem gleichen Betrag in genau entgegengesetzter Richtung zum Pfannenpol hin zu verschieben, und das Werkzeug mit dem größten Schneidkantenwinkel (z.B. 42°) in genau umgekehrter Weise in Richtung zum Pfannenpol hin und gleichzeitig mit dem gleichen Betrag die entsprechende Konturbeschreibung zum Pfannenäquator hin jeweils um den gleichen Betrag zu verschieben.

Die mit der Erfindung vorgeschlagene Sehnenkorrektur ist vorteilhaft, wenn die mit den Werkzeugen abzufahrenden Bahnen in Ermangelung anderer Programmierungsmöglichkeiten der Maschinensteuerung quasi aus vielen kleinen Kegelgewindeabschnitten zusammengesetzt sind. Ohne Berücksichtigung der Sehnenkorrektur liegen die Anfangs- bzw. Endpunkte dieser Kegelgewindeabschnitte auf dem vom Nenndurchmesser der Schraubpfanne gebildeten Kugelmantel, wobei die so gebildeten Sehnen diesen Kugelmantel je nach Länge des jeweiligen Kegelabschnitts mehr oder weniger unterschneiden. Dieser Unterschnitt wird mittels der vorgeschlagenen Sehnenkorrektur dadurch verringert, daß die genannten Sehnen radial vom Pfannenmittelpunkt weg geringfügig nach außen hin verschoben werden, wobei die von den Kegelgewindeabschnitten gebildete eckige Bahn mehr oder weniger gemittelt auf dem vom Nenndurchmesser der Schraubpfanne im Schnitt gebildeten Kreisbogen zu liegen kommt. Absolut gesehen, ist die Sehnenkorrektur relativ klein. Sie nimmt auch mit steigender Zahl der angesetzten Kegelgewindeabschnitte ab, bzw. wird nahezu bedeutungslos bei einer relativ feinmaschig aufgelösten Programmierung.

Gemäß der Erfindung wird ferner eine Dachkantenkorrektur vorgeschlagen. Diese wird dadurch erforderlich, daß im Gewindegrund aufgrund der vorgeschlagenen Bearbeitungsweise Dachkanten gebildet sind, solange zwei Werkzeuge am Fertigschnitt des Gewindegrundes beteiligt sind. Diese Dachkanten verschwinden vollständig, wenn der Tangentenwinkel des Gewindesgrundes exakt einem der Schneidenwinkel der Werkzeuge entspricht. Sie erreichen andererseits ihre maximale Höhe, wenn dieser Tangentenwinkel genau zwischen zwei Schneidenwinkeln der Werkzeuge liegt. Werden nun die Eckenradien der Werkzeuge genau entlang der vom Nenndurchmesser der Pfanne gebildeten Kugelmantel bewegt, so ragen diese Dachkanten nach außen vor. Mit der Dachkantenkorrektur werden die Schneidwerkzeuge so radial zum Pfannenmittelpunkt hin korrigiert, daß ein fließender Übergang zwischen dem unkorrigierten Bereich der vom jeweiligen Werkzeug beschriebenen Bahn und dem Punkt der maximalen Korrektur im Bereich der maximalen Dachkantenhöhe erzielt wird. Es wird vorteilhaft sein, den Betrag dieser Dachkantenkorrektur so anzusetzen, daß im Bereich der jeweils maximalen Dachkantenhöhe die Dachkante nicht mehr als 0,1 Millimeter gegenüber der vom Nenndurchmesser der Schraubpfanne gebildeten Kugelmantel vorsteht. Der absolute Betrag der Dachkantenkorrektur nimmt mit steigender Anzahl der Werkzeuge und der damit verbesserten Auflösung der Winkelschritte ab.

Der Vorteil des erfindungsgemäßen Herstellungsverfahrens liegt darin, daß die zerspanungstechnisch bisher nicht gelöste Aufgabe nicht nur gelöst, sondern auch auf unkomplizierte und preiswerte Weise gelöst wird. Das gilt insbesondere dann, wenn die Herstellung drehtechnisch mittels einer CNC-Maschine erfolgt.

Die erfindungsgemäße Hüftgelenkpfanne wird daher vorzugsweise drehtechnisch hergestellt. Es wird empfohlen, die Gewinderille zunächst mit einem Stecher grob vorzuschruppen. Bei der Fertigbearbeitung z.B. mit drei Stechwerkzeugen sind deren Stirnwinkel gemäß der Erfindung vorzugsweise nahezu gleichmäßig entsprechend dem zu bearbeitenden Winkelbereich gestaffelt (z.B. 0°, 21°, 40°). Von den drei Werkzeugen muß mindestens eines die rechte, bzw. ein zweites die linke Gewindeflanke schneiden. Gewöhnlich liegen diese Flankenwinkel zwischen 6° und 15°. Fur die normale Aufspannung (Kugelmittelpunkt zum Futter) wird empfohlen, das Stechwerkzeug mit dem größten Stirnwinkel gleichzeitig für die Bearbeitung der rechten Flanke der Gewinderille einzusetzen, während umgekehrt empfohlen wird, das Stechwerkzeug mit dem kleinsten Stirnwinkel (z.B. 0°) zum Schneiden der linken Flanke der Gewinderille zu benutzen. Für die Bearbeitung auf einer sogenannten zweiachsigen CNC-Drehmaschine werden die Werkzeuge nacheinander aufgerufen und das Gewinde abgefahren. Dabei muß für jedes der Werkzeuge eine eigene Konturbeschreibung (Beschreibung der Gewindebahn) eingegeben und mit der Werkzeugbeschreibung in Einklang gebracht werden. Die Konturbeschreibung muß die gewünschten erfindungsgemäßen Bahnkorrekturen enthalten. Es ist ferner zu berücksichtigen, daß bei der Programmierung der Gewindeschneidzyklen die jeweiligen Start- bzw. Endpunkte für sämtliche Werkzeuge identisch sein müssen, damit jedes Werkzeug in einer exakt synchronisierten Position in der Gewinderille verfahren wird.

Die Erfindung soll im folgenden anhand der Zeichnungsfiguren näher erläutert werden.

Fig. 1 zeigt den Axialschnitt eines Ausführungsbeispiels der erfindungsgemäßen Hüftgelenkpfanne in einer abgebrochen gezeichneten Darstellung.

In Fig. 2 ist anhand eines geometrischen Schemas die Entstehung einer unkorrigierten Bahn einer einzelnen Schneidplatte während des Abfahrens der Kugelkontur dargestellt.

Fig. 3 zeigt eine Abwicklung des Gewindegrundes in stark vereinfachter Darstellung.

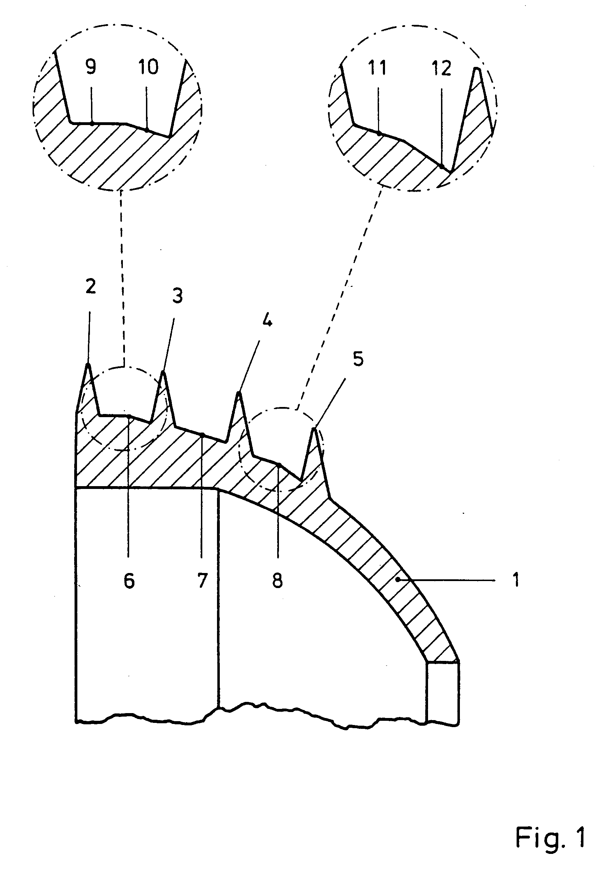

Bei dem in Fig. 1 gezeigten Ausführungsbeispiel handelt es sich um die metallische Schale einer erfindungsgemäßen Hüftgelenkpfanne, wobei sowohl die nach hinten verlaufenden Kanten der Gewindezähne, als auch das erforderliche Inlett aus Kunststoff aus Gründen der Vereinfachung und der besseren Übersichtlichkeit weggelassen wurden.

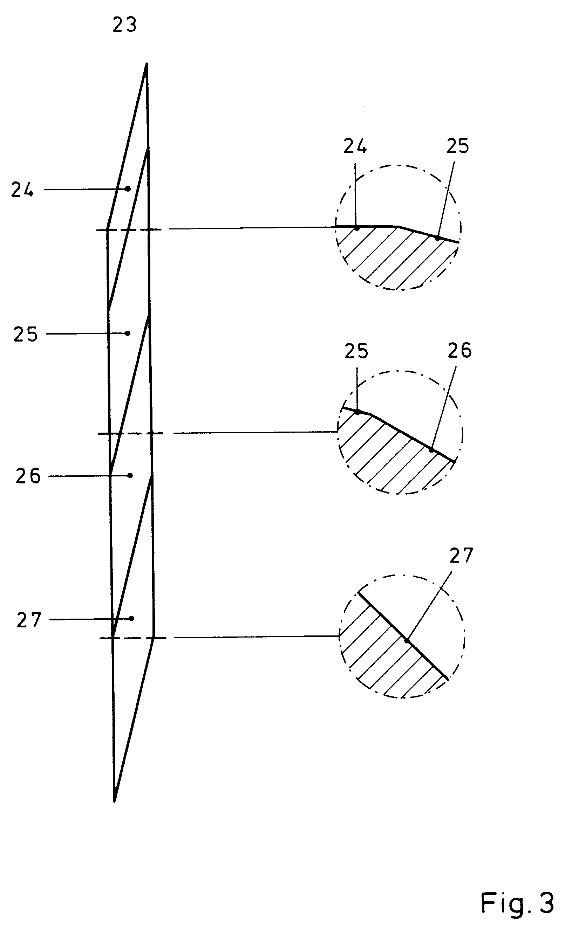

Die Hüftgelenkpfanne 1 besitzt ein Gewinde, von welchem die vier Gewindzähne 2,3,4,5 zwischen sich eine Gewinderille mit jeweils einem Gewindegrund 6,7,8 unterschiedlicher Form bilden. Sowohl der Gewindegrund 6 als auch der Gewindegrund 8 sind oben vergrößert herausgezeichnet. Das gezeichnete Gewinde zeigt das Ergebnis einer Fertigbearbeitung des Gewindegrundes mit drei verschiedenen Werkzeugen 16 unterschiedlicher Schneidkantenwinkel von 0°, 17° und 34°. In der Vergößerung des Gewindegrundes 6 ist gut zu erkennen, daß der linke Bereich 9 von dem Werkzeug mit dem kleinsten Schneidkantenwinkel (0°), und der rechte Bereich 10 von dem Werkzeug mit dem mittleren Schneidkantenwinkel (17°) bearbeitet worden ist. Im Gegensatz dazu macht die Vergrößerung des Gewindegrundes 8 deutlich, daß nunmehr der linke Bereich 11 von dem Werkzeug mit dem mittleren Schneidkantenwinkel (17°), und der rechte Bereich 12 von dem Werkzeug mit dem größten Schneidkantenwinkel (34°) bearbeitet worden ist. Zwischen den aufgrund der unterschiedlichen Gewindegrundwinkel gebildeten Teilflächen ist jeweils eine Dachkante gebildet, wobei jedoch deren Lage sich kontinuierlich umlaufend Verschiebt, ein Effekt, welcher der zweidimensionalen Darstellung nicht zu entnehmen, aber aus Figur 3 ersichtlich ist. In Figur 1 liegt die mittlere Gewinderille mit dem Gewindegrund 7 so, daß sie im wesentlichen mit dem Werkzeug mit dem mittleren Schneidkantenwinkel (17°) bearbeitet ist. Der geringe Materialabtrag der beiden anderen Werkzeuge in der linken bzw. rechten unteren Ecke des Gewindegrundes ist derart klein, daß er in der Darstellung nicht sichtbar ist. Anhand der Zeichnungsfigur wird jedoch deutlich, wie gut mittels des erfindungsgemäßen Verfahrens die Nachbildung des im Axialschnitt kreisbogenförmigen Verlaufs des Gewindesgrundes realisierbar ist.

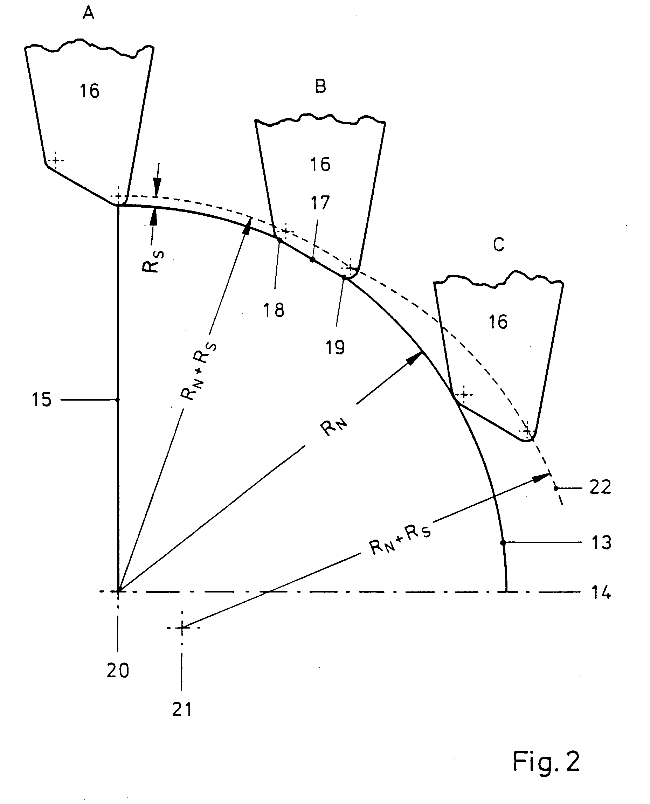

Das in Fig. 2 dargestellte geometrische Schema soll die Entstehung einer einzelnen zunächst unkorrigierten Bahn 22 eines der Werkzeuge 16 während des Abfahrens der Kontur zwecks Ausführung des erfindungsgemäßen Verfahrens anhand eines Beispiels veranschaulichen. Es ist ein voll ausgezogener Kreisbogen 13 als Viertelkreis mit dem Radius R_{N} dargestellt, welcher dem vom Nenndurchmesser gebildeten Kugelmantel der Halbseite einer Hüftgelenkpfanne 14 entspricht. Die Mittelachse der Hüftgelenkpfanne 14 ist in üblicher Weise strich-punktiert gezeichnet, während eine durchgezogene Linie 15 den Pfannenäquator verdeutlichen soll. Es ist ferner ein abgebrochen dargestelltes Bearbeitungswerkzeug 16 in drei verschiedenen Positionen (A,B,C) während des letzten Schnitts der Bearbeitung dargestellt. Die den Gewindegrund bearbeitende Schneide 17 besitzt einen Stirnwinkel von 20°. Die beiden Ecken 18,19 der Schneide 17 sind mit einem Radius R_{S} versehen. Aufgrund der vorgeschlagenen Bahn 22 des Werkzeugs 16, wobei die in Richtung zum Kugelmittelpunkt 20 der Hüftgelenkpfanne 14 jeweils am weitesten vorstehende Ecke der Schneide 17 entlang der Kugelkontur verfahren wird und damit zunächst zwischen den Positionen A und B die rechte Ecke 19 und zwischen den Positionen B und C die linke Ecke 18 des Werkzeugs 16 auf dieser Kugelkontur bewegt wird, ergibt sich für die gestrichelt eingetragene Bahn 22 ein aus zwei Halbbögen mit dem jeweiligen Radius R_{N}+R_{S} zusammengesetzter Verfahrweg. Während der Drehpunkt des ersten Halbbogens mit dem Kugelmittelpunkt 20 der Pfanne übereinfallt, ist für den zweiten Halbbogen ein neuer Drehpunkt 21 gebildet. Die relative Position dieser beiden Drehpunkte 20,21 zueinander ist mit der relativen Position der beiden Eckenradienmittelpunkte des Werkzeugs 16 identisch. Daraus ergibt sich, daß für jedes andere Bearbeitungswerkzeug der entweder einzige, bzw. der jeweils zweite Drehpunkt entsprechend angepaßt festzulegen ist. Die gemäß der Erfindung weiter vorgeschlagenen Korrekturen zur noch besseren Anpassung des spanend hergestellten Gewindegrundes an den vom Nenndurchmesser der Hüftgelenkpfanne gebildeten Kugelmantel sind in der Fig. 2 nicht berücksichtigt.

Der in Figur 3 als Abwicklung dargestellte Gewindegrund 23 ist stark vereinfacht gezeichnet und dient lediglich der verständlicheren Erläuterung seines Aufbaus. Aus dieser Darstellungsweise ist gut ersichtlich, daß er aus streifenförmigen schräg zur Abwicklung verlaufenden Teilflächen 24,25, 26,27 zusammengesetzt ist, die an den Dachkanten zusammenstoßen. Letzteres ist in den vergrößert herausgezogenen Querschnittsausschnitten dargestellt, die denen in Figur 1 im Prinzip entsprechen. Die Winkel zwischen den Teilflächen 24,25,26,27 ergeben sich, wie schon dargelegt, aus den Schneidkantenwinkeln der sie bearbeitenden Werkzeuge 16. Durch die Teilflächen ist der Gewindegrund der sphärischen äußeren Kontur der Hüftgelenkpfanne 1 weitestgehend angeglichen.

## Patentansprüche

1. Einschraubbare Hüftgelenkpfanne mit sphärischer äußerer Kontur, welche mit einem selbstschneidenden Gewinde zur zementfreien Verankerung im Acetabulum versehen ist, dadurch gekennzeichnet, daß der Gewindegrund in seiner Abwicklung aus streifenförmigen, in der Ebene der Abwicklung parallel zueinander verlaufenden Teilfächen besteht, die zwischen sich jeweils eine stumpfwinklige und schräg zur Erstreckung der Abwicklung verlaufende Dachkante ausbilden, wobei diese Teilflächen im Hüftgelenkpfannen-Axialquerschnitt gerade oder konvex gekrümmte Querschnittsbereiche (9, 10, 11, 12) aufweisen, die im HÜftgelenkpfannen-Axialquerschnitt schräg zu anderen Querschnittsbereichen (9, 10, 11, 12) verlaufen, und wobei diese den Gewindegrund bildenden Teilflächen der sphärischen äußeren Kontur der Hüftgelenkpfanne (1) weitestgehend angeglichen sind.

2. Verfahren zur spanenden Herstellung der Hüftgelenkpfanne nach Anspruch 1, dadurch gekennzeichnet, daß das Gewinde mit verschiedenen Werkzeugen (16) mit in bezug auf den Gewindegrund (6, 7, 8) unterschiedlicher Schneidkantengeometrie auf unterschiedlichen Bahnen bearbeitet wird, wobei jeweils der in Richtung zum Mittelpunkt der vom Nenndurchmesser der Hüftgelenkpfanne gebildeten Halbkugel am weitesten vorstehende Punkt der Schneidkante im wesentlichen parallel zu bzw. auf dem Mantel dieser Halbkugel bewegt wird, und wobei die den Gewindegrund bildenden Teilflächen dem Oberflächenverlauf einer dem Nenndurchmesser der Hüftgelenkpfanne entsprechenden Halbkugel möglichst weitgehend angepaßt sind.

3. Verfahren nach Anspruch 2, dadurch gekennzeichnet, daß das Gewinde mit mindestens drei Werkzeugen (16) mit in bezug auf den Gewindegrund (6, 7, 8) unterschiedlicher Schneidkantengeometrie bearbeitet wird, wobei die Schneidkantenwinkel der den Gewindegrund bearbeitenden Schneidkanten (17) der Werkzeuge (16) im Rahmen des Tangentenwinkels des vom Gewinde überstrichenen Halbkugelbereichs abgestuft sind.

4. Verfahren nach Anspruch 3, dadurch gekennzeichnet, daß die den Gewindegrund bearbeitende Schneidkante (17) eines Werkzeugs (16) im wesentlichen dem kleinsten Tangentenwinkel, die den Gewindegrund bearbeitende Schneidkante eines weiteren Werkzeugs (16) im wesentlichen dem halben Tangentenwinkel und die Schneidkante eines dritten Werkzeugs (16) im wesentlichen dem größten Tangentenwinkel des vom Gewinde überstrichenen Halbkugelbereichs entspricht.

5. Verfahren nach einem der Ansprüche 2 bis 4, dadurch gekennzeichnet, daß die zwischen den Mittelpunkten der Radien der Ecken (18, 19) der einzelnen Werkzeuge (16) in axialer Richtung des Gewindes gemessene Breite im wesentlichen gleich ist.

6. Verfahren nach einem der vorhergehenden Ansprüche 2 bis 5, dadurch gekennzeichnet, daß als Radius für die Festlegung der von den einzelnen Werkzeugen (16) abzufahrenden Bahnen (22) die Summe aus dem Nennradius (R_{N}) der Hüftgelenkpfanne (1) und dem Schneidenradius (R_{S}) des jeweiligen Werkzeugs (16) herangezogen wird, und gleichzeitig als Bezugspunkt in der Werkzeugbeschreibung der Mittelpunkt des Schneidenradius (R_{S}) einer Ecke (18, 19) des Werkzeugs (16) benutzt wird.

7. Verfahren nach einem der vorhergehenden Ansprüche 2 bis 6, dadurch gekennzeichnet, daß das Werkzeug (16) mit dem kleinsten Schneidkantenwinkel mittels einer Werkzeugkorrektur geringfügig zum Pfannenäquator hin und gleichzeitig die Konturbeschreibung in Gestalt der abzufahrenden Bahn (22) mit dem gleichen Betrag zum Pfannenpol hin verschoben wird, und das Werkzeug (16) mit dem größten Schneidkantenwinkel in umgekehrter Weise in Richtung zum Pfannenpol hin und gleichzeitig mit dem gleichen Betrag die entsprechende Konturbeschreibung zum Pfannenäquator hin verschoben wird.

8. Verfahren nach einem der vorhergehenden Ansprüche 2 bis 7, dadurch gekennzeichnet, daß die von den einzelnen Werkzeugen (16) abzufahrenden und auf Abschnitten von Kreisbögen liegenden Bahnen (22) nachgebildet werden, indem zuerst auf diesen Kreisbogenabschnitten liegende Punkte beschrieben und damit Sehnen gebildet werden, und dann die so entstehenden Sehnen jeweils radial vom Pfannenmittelpunkt (20) weg geringfügig nach außen verschoben werden.

9. Verfahren nach einem der vorhergehenden Ansprüche 2 bis 8, dadurch gekennzeichnet, daß die von den Werkzeugen (16) beschriebenen Bahnen (22) im Axialschnitt der Hüftgelenkpfanne (1) abweichend von exakten Kreisbögen in ihrem radialen Abstand von der Mittelachse (14) der Hüftgelenkpfanne (1) korrigiert werden, wobei mittels der Größe dieser Korrektur die im Gewindegrund ausgebildeten Dachkanten nach der Fertigbearbeitung gegenüber dem vom Nenndurchmesser der Hüftgelenkpfanne gebildeten Halbkugelmantel nicht mehr als 0,1 mm nach außen vorstehen.

## Claims

1. Screw-on hip-joint socket having a spherical outer shape, which socket is provided with a self-tapping thread for cement-free anchoring in the acetabulum, **characterized in that** in its development the thread bottom consists of strip-shaped surface portions extending in the plane of the development parallel to each other, which surface portions form respective roof edges between each other having an obtuse angle and extending oblique to the extension of the development, said surface portions comprising, in the axial section of the hip-joint socket, straight or convex curved section portions (9, 10, 11, 12), which in the axial section of the hip-joint socket extend oblique to other section portions (9, 10, 11, 12), and said surface portions forming said thread bottom being matched to the spherical outer shape of the hip-joint socket (1) to the maximum extent possible.

2. A process for the machine production of the hip-joint socket as defined in claim 1, **characterized in that** the thread is machined with tools (16) with different cutting edge geometries relative to the thread bottom (6, 7, 8) on different paths such that the point of the particular cutting edge which projects furthest in the direction of the mid point of the hemispherical surface formed by the nominal diameter of the hip-joint socket is moved essentially parallel to or on this hemispherical surface.

3. A process as defined in claim 2, **characterized in that** the thread is machined with at least three tools (16) having cutting edge geometries that differ relative to the thread bottom (6, 7, 8), the cutting edge angle of the cutting edges (17) of the tools (16) that machine the thread bottom being graduated within the frame work of the tangent angle and of the hemispherical area that is swept by the thread.

4. A process as defined in claim 3, **characterized in that** the cutting edge (17) of a tool (16) that machines the thread bottom corresponds essentially to the smallest tangent angle, the cutting edge of a further tool (16) that machines the thread bottom corresponds essentially to the half-tangent angle and the cutting edge of a third tool (16) corresponds essentially to the greatest tangent angle of the hemispherical area that is swept by the thread.

5. A process as defined in one of the claims 2 - 4, **characterized in that** the width of the individual tools, measured between the center points of the radii of the corners (18, 19) in the axial direction of the thread, is essentially equal.

6. A process as defined in one of the preceding claims 2 - 5, **characterized in that** the sum of the nominal radius (R_{N}) of the hip-joint socket (1) and the cutting radius (R_{S}) of the particular tool (16) is used as the radius for establishing the paths (22) to be followed by the individual tools (16), and the center point of the cutting radius (R_{S}) of the corner (18, 19) of the tool (16) is used simultaneously as a reference point in the tool description.

7. a process as defined in one of the preceding claims 2 - 6, **characterized in that** the tool (16) with the smalles cutting edge angle is moved by means of a tool correction slightly towards the socket equator and simultaneously the shape description in the form of the path to be followed (22) is shifted by the same amount towards the socket pole, and the tool (16) with the greatest cutting edge angle is moved in the opposite way in the direction of the socket pole and simultaneously the corresponding shape description is shifted to the socket equator by the same amount.

8. A process as defined in one of the preceding claims 2 - 7, **characterized in that** the paths (22) that are to be followed by individual tools (16) and which lie on sections of archs of a circle are formed by describing first the points lying on these sections of archs of a circle and then forming chords therebetween, and then shifting the chords radially slightly outwards away from the mid-point (22) of the sokket.

9. A process as defined in one of the preceding claims 2 - 8, **characterized in that** the paths (22) described by the tools (16) in the axial section of the hip-joint socket are corrected so as to deviate from the exact arch sections in the radial distance from the middle axis (14) of the hip-joint socket (1), by means of the amount of this correction the roof edges formed in the thread bottom after machining projecting no more than 0.1 mm outwards relative to the hemispherical surface formed by the nominal diameter of the hip-joint socket.

## Revendications

1. Prothèse acétabulaire a visser a contour extérieur sphérique qui est pourvue d'un filet autotaraudeur pour l'ancrage sans ciment dans l'acétabulum, caractérisée en ce que le fond de filet est constitué, dans son développement, par des surfaces partielles en forme de bande, qui sont parallèles l'une à l'autre dans le plan du développement, surfaces partielles qui forment entre elles respectivement une arête supérieure à angle obtus, oblique par rapport à l'extension du développement, ces surfaces partielles présentant, dans la section axiale de l'acétabulum, des zones de section (9, 10, 11, 12), droites ou à courbure convexe, qui sont obliques par rapport à d'autres zones de section (9, 10, 11, 12) dans la section axiale de la prothèse acétabulaire, et ces surfaces partielles qui forment le fond de filet étant extrêmement adaptées au contour extérieur sphérique de la prothèse acétabulaire (1).

2. Procédé pour la fabrication par enlèvement de copeaux de la prothèse acétabulaire selon la revendication 1, caractérisé en ce que le filet est usiné sur différentes trajectoires avec différents outils (16) avec une géométrie de tranchant différente par rapport au fond de filet (6, 7, 8), le point du tranchant, qui est respectivement saillant le plus en avant en direction du centre de l'hémisphère formée par le diamètre nominal de la prothèse acétabulaire, étant déplacé essentiellement parallèlement a ou sur la surface latérale de cette hémisphère et les surfaces partielles formant le fond de filet étant adaptées le plus possible à l'allure de la surface d'une hémisphère qui correspond au diamètre nominal de la prothèse acétabulaire.

3. Procédé selon la revendication 2, caractérisé en ce que le filet est usiné avec au moins trois outils (16) avec une géométrie de tranchant différente par rapport au fond de filet (6, 7, 8), les angles de tranchant des tranchants (17) des outils qui usinent le fond de filet étant graduels dans le cadre de l'angle de la tangente de la zone de l'hémisphère balayée par le filet.

4. Procédé selon la revendication 3, caractérisé en ce que le tranchant (17) d'un outil (16) qui usine le fond de filet correspond essentiellement au plus petit angle de tangente, le tranchant d'un autre outil (16) qui usine le fond de filet correspond essentiellement à la moitié de l'angle de tangente et le tranchant d'un troisième outil (16) correspond essentiellement au plus grand angle de tangente de la zone de l'hémisphère balayée par le filet.

5. Procédé selon l'une des revendications 2 à 4, caractérisé en ce que la largeur mesurée entre les centres des rayons des coins (18, 19) des différents outils (16) dans le sens axial du filet est essentiellement égale.

6. Procédé selon l'une des revendications précédentes 2 à 5, caractérisé en ce que l'on prend comme rayon pour la définition des trajectoires (22) devant être parcourues par les différents outils (16) la somme du rayon nominal (R_{N}) de la prothèse acétabulaire (1) et du rayon de coupe (R_{S}) de l'outil respectif (16) et que le centre du rayon de coupe (R_{S}) d'un coin (18, 19) de l'outil (16) est utilisé simultanément comme point de référence dans la description de l'outil.

7. Procédé selon l'une des revendications précédentes 2 à 6, caractérisé en ce que l'outil (16) avec le plus petit angle de tranchant est déplacé légèrement vers l'équateur de la prothèse par une correction d'outil et simultanément la description du contour sous forme de la trajectoire (22) à parcourir est déplacée de la même valeur vers le pôle de la prothèse, et l'outil (16) avec le plus grand angle de tranchant est déplacé dans le sens inverse en direction du pôle de la prothèse et simultanément la description du contour correspondant est déplacée de la même valeur vers l'équateur de la prothèse.

8. Procédé selon l'une des revendications précédentes 2 à 7, caractérisé en ce que les trajectoires (22) devant être parcourues par les différents outils (16) et se trouvant sur des sections d'arc de cercle sont reproduites en décrivant tout d'abord des points qui se trouvent sur ces sections d'arc de cercle et en formant ainsi des cordes et en déplaçant les cordes ainsi obtenues légèrement vers l'extérieur respectivement radialement en s'écartant du centre (20) de la prothèse.

9. Procédé selon l'une des revendications précédentes 2 à 8, caractérisé en ce que les trajectoires (22) décrites par les outils (16) dans la coupe axiale de la prothèse acétabulaire (1) sont corrigées en différant des arcs de cercle exacts quant à leur écart axial par rapport à l'axe central (14) de la prothèse acétabulaire (1), les arêtes supérieures formées dans le fond de filet au moyen de la grandeur de cette correction ne faisant pas saillie, après achèvement de l'usinage, de plus de 0,1 mm vers l'extérieur par rapport à l'enveloppe d'hémisphère formée par le diamètre nominal de la prothèse acétabulaire.
